# EUROPÄISCHE PATENTSCHRIFT

(11) **EP 0 669 380 B1**
(45) Veröffentlichungstag und Bekanntmachung des Hinweises auf die Patenterteilung: **28.03.2001**
(21) Anmeldenummer: 95810083.6
(22) Anmeldetag: 08.02.1995
(51) Int. Cl.: C09B 29/01, C07C 245/20, C07C 245/08

(54) **Verfahren zur Herstellung von Azofarbstoffen**
Process for the preparation of azo dyes
Procédé de préparation de colorants azoiques

(30) Priorität: 21.02.1994 DE 4405469
(43) Veröffentlichungstag der Anmeldung: 30.08.1995
(73) Patentinhaber: Ciba Specialty Chemicals Holding Inc., 4057 Basel (CH)
(72) Erfinder: Langfeld, Dr. Horst, D-79639 Grenzach-Wyhlen (DE); Mauser, Dr. Herbert, D-79639 Grenzach-Wyhlen (DE); Haarburger, Karl-Friedrich,, D-79541 Lörrach (DE)

(56) Entgegenhaltungen:
- EP-A- 0 001 236
- EP-A- 0 003 656
- EP-A- 0 184 555
- EP-A- 0 209 492
- EP-A- 0 220 130
- DE-B- 1 231 251
- FR-A- 2 320 336
- FR-A- 2 331 604
- DYES AND PIGMENTS, Bd. 5, Nr. 1, 20.Oktober 1984 - 1984, GREAT YARMOUTH, GB, Seiten 49-64, XP002019061 J. SZADOWSKI ET. AL.: "Relationships Between the Structure of Nitrodiphenylamine Derived ..."
- CHEMICAL ABSTRACTS, vol. 96, no. 24, 14.Juni 1982 Columbus, Ohio, US; abstract no. 201256u, C. PRZYBYLSKI ET. AL.: "Azo Dyes" Seite 85; Spalte 1; XP002019062 & PL 108 921 A

## Beschreibung

Die vorliegende Erfindung betrifft ein Verfahren zur Herstellung von Azofarbstoffen durch Diazotierung eines aromatischen Amins und Kuppeln mit einer Kupplungskomponente, in dem die Diazotierung kontinuierlich durchgeführt wird.

Die Diazotierung von aromatischen Aminen, insbesondere von Amino-diphenylaminen und gewissen heterocyclischen Aminen, wird von Schwierigkeiten begleitet, zu deren Überwindung man in der Praxis meist die Methode der indirekten Diazotierung anwendet, wobei man eine wässrig-alkalische Lösung des Gemisches aus Amin und Alkalinitrit von ca. 80 °C in eine eisgekühlte wässrige Lösung einer Mineralsäure einträgt. Nitrit wird dabei in ca. 5 bis 7 % Überschuss eingesetzt.

Auch diese Arbeitsweise ist jedoch mit Nachteilen behaftet. Da die Reaktion bei ca. 0 °C beginnt, fällt das Amin in Form von klebrigen Agglomeraten aus, deren vollständige Diazotierung dann, wegen erschwertem Reaktandenkontakt nur nach mehrstündigem Rühren bei ca. 60 bis 65 °C gelingt. Dies bringt teilweise Zersetzung der Diazoverbindung, eine grosse Belastung des Reaktionskessels infolge der starken Temperaturunterschiede und der konzentrierten NOₓ-Atmosphäre bei erhöhter Temperatur sowie eine Abluftbelastung durch die NOₓ-Emission mit sich. Für die Diazotierung sowie nach Beendigung dieser zur Vorbereitung der Kupplung müssen grosse Mengen Eis zugegeben werden, was zu unerwünschter starker Verdünnung der Reaktionslösung und zu sehr ungünstigen Raum/Zeit-Ausbeuten führt.

Gemäss der EP-A-0 001 236 wird ein Verfahren zur kontinuierlichen Diazotierung von aromatischen Aminen vorgeschlagen, bei dem ein Teilstrom des Gemisches aus Amin und Nitrit durch einen Analysator geleitet wird, wo der Nitritüberschuss gemessen wird. An Hand dieses Messwertes wird dann die Nitritzugabe in der Vorlage gesteuert. Diese Arbeitsweise erfordert jedoch eine aufwendige Vorrichtung zur Analyse des Nitritüberschusses. Hinzu kommt, dass die Reaktionssteuerung über den Nitritüberschuss im Falle der Amino-diphenylamine wegen der sekundär eintretenden N-Nitrosierung nicht zuverlässig ist.

Es wurde nun gefunden, dass man aromatische Amine der nachstehend angegebenen Formel (1) auf einfache Art diazotieren kann, wenn man die Diazotierung kontinuierlich bei ca. 35 bis 65 °C durchführt. Überraschenderweise ist die Diazoverbindung ausreichend stabil und eine aufwendige Steuerung des Nitritüberschusses ist nicht erforderlich, da überschüssiges Nitrit anschliessend leicht zerstört werden kann, z.B. durch Zugabe von Sulfaminsäure. Durch nachfolgende übliche Kupplung kann man dann Farbstoffe von hervorragender Qualität und in verbesserter Ausbeute herstellen. Die Farbstoffe enthalten keine mit bekannten Methoden feststellbaren sek.-NO-Gruppen mehr. Auch die Qualitätskonstanz der Farbstoffe ist überraschend hoch. Die Nuance schwankt nur noch geringfügig innerhalb der akzeptierten Standardkonformität, so dass kostspielige Korrekturmassnahmen in der Endstandardisierstufe nicht mehr nötig sind, die bei den bekannten Verfahren nicht zu umgehen sind, da dort die Nuance der erhaltenen Farbstoffe stark schwankt, auch bei nur minimalen, unvermeidbaren Abweichungen der Reaktionsbedingungen, wie Temperatur, pH-Wert, Rührgeschwindigkeit etc. Ein weiterer Vorteil des erfindungsgemässen Verfahrens sind die Schonung der Reaktionsgefässe infolge geringerer Temperaturunterschiede und niedrigerem NOₓ-Gehalt sowie die energiesparende Prozessführung bei gleichzeitig hoher Raum / Zeit-Ausbeute.

Gegenstand der vorliegenden Erfindung ist ein Verfahren zur Herstellung von Azofarbstoffen, ausgehend von einem Amin der Formel 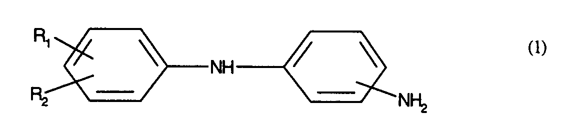 worin
R₁ Wasserstoff oder Nitro und
R₂ Wasserstoff, Sulfo, C₁-C₄-Alkyl oder C₁-C₄-Alkoxy bedeutet,
oder ausgehend von einem Amin. ausgewählt aus der Gruppe, bestehend aus 2-Aminobenzthiazol, 6-Nitro-2-aminobenzthiazol, 6-Benzoyl-2-aminobenzthiazol, 6-Methyl-2-aminobenzthiazol, 6-Methoxy-2-aminobenzthiazol, 2-Aminotriazol, 5-Methylsulfonyl-2-aminotriazol, 5-Nitro-2-aminotriazol, 3-Amino-1,2,4-triazol, 3-Aminoindazol, 5-Aminoindazol, 4-Aminochinolin und Aminobenzimidazol-Derivaten,
dadurch gekennzeichnet, dass man bei 35 bis 65 °C das Amin, ein Alkalinitrit und eine Mineralsäure kontinuierlich zu der Diazoverbindung umsetzt, wobei man das Alkalinitrit in 3 bis 15 %igem Überschuss verwendet, und anschliessend die Diazoverbindung mit einer Kupplungskomponente kuppelt.
R₁ bedeutet vorzugsweise Nitro, insbesondere Nitro in p-Stellung zur NH-Gruppe.

Die bevorzugte Bedeutung von R₂ ist Wasserstoff oder vor allem Sulfo

Ganz besonders bevorzugt verwendet man ein Amin der Formel 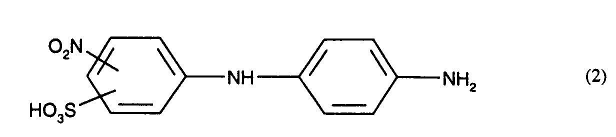 und insbesondere bevorzugt ist die Verwendung von 4'-Amino-4-nitrodiphenylamin-2-sulfosäure als aromatisches Amin.

Die genannten Amine werden als wässrige Lösungen oder Suspensionen eingesetzt, vorzugsweise werden sie durch Anschlämmen in Wasser und Zugabe einer Base, wie z.B. KOH oder NaOH und Erhitzen auf 40 bis 90 °C vollständig gelöst.

Als Alkalinitrit setzt man z.B. Kalium- oder vor allem Natriumnitrit ein und als Mineralsäure verwendet man z.B. Schwefelsäure oder vor allem Salzsäure.

Das Alkalinitrit wird in 3 bis 15 %igem Überschuss, bezogen auf Amin der Formel (1), eingesetzt, vorzugsweise in 5 bis 10 %igem Überschuss.

Pro Mol Amin setzt man vorzugsweise etwa 2,5 Mol Mineralsäure ein.

Die Diazotierung erfolgt in einem handelsüblichen kontinuierlichen Durchflussreaktor, welcher vorzugsweise mit einem Mischorgan für sehr intensive Vermischung der Komponenten ausgestattet ist. Man geht z.B. so vor, dass man Amin, Alkalinitrit und Mineralsäure z.B. über drei getrennte Leitungen synchron in den erforderlichen Mengen zudosiert. Man kann aber auch zwei der Komponenten, beispielsweise Amin und Alkalinitrit vor dem Eintritt in den Durchflussreaktor miteinander vermischen.

Die Reaktionstemperatur liegt zwischen 35 und 65 °C, vorzugsweise zwischen 40 und 55 °C.

Die Verweilzeit in dem Durchflussreaktor hängt u.a. von der Art des aromatischen Amines und der Temperatur ab. Im allgemeinen liegt sie zwischen etwa 0,1 und 10 Minuten, vorzugsweise zwischen etwa 0,5 und 5 Minuten.

Nach Verlassen des Durchflussreaktors wird die Lösung der Diazokomponente gegebenenfalls gekühlt und auf übliche Weise auf eine Kupplungskomponente gekuppelt, vorzugsweise diskontinuierlich.

Es ist bevorzugt, in dem Reaktionsgemisch nach Verlassen des Durchflussreaktors eventuell noch vorhandenes Nitrit vor der anschliessenden Kupplung zu zerstören. Dies geschieht auf bekannte Art, beispielsweise durch Zugabe von Sulfaminsäure oder Harnstoff.

Die Kupplung erfolgt auf übliche Art und Weise mit den bei der Herstellung von Azofarbstoffen üblichen Kupplungskomponenten. Da die Amine der Formel (1) vor allem bei der Herstellung von Lederfarbstoffen Verwendung finden, kommen als Kupplungskomponenten insbesondere Phenole, Naphthole, Aniline und Naphthylamine in Betracht, welche noch weitere Substituenten, wie z.B. Nitro, Sulfo, Alkyl, Alkoxy oder Arylazo, tragen können. Ebenfalls geeignet sind die für Lederfarbstoffe gebräuchlichen Gelbholzextrakte, die Polyhydroxyverbindungen, wie z.B. Maclurin, Morin oder Quercetin enthalten.

Anschliessend wird der Farbstoff auf übliche Weise isoliert, z.B. durch Aussalzen oder Eindampfen in geeigneten Apparaturen.

Das erfindungsgemässe Verfahren zur Herstellung von Azofarbstoffen eignet sich neben der Herstellung von Azofarbstoffen, ausgehend von Amino-diphenylaminen als Diazokomponente, auch hervorragend zur Herstellung von Azofarbstoffen mit heterocyclischen Diazokomponenten. Als solche kommen z.B. in Frage: 2-Aminobenzthiazol, 6-Nitro-2-aminobenzthiazol, 6-Benzoyl-2-aminobenzthiazol, 6-Methyl-2-aminobenzthiazol, 6-Methoxy-2-aminobenzthiazol, 2-Aminotriazol, 5-Methylsulfonyl-2-aminotriazol, 5-Nitro-2-aminotriazol, 3-Amino-1,2,4-triazol, 3-Aminoindazol, 5-Aminoindazol, 4-Aminochinolin sowie Aminobenzimidazol-Derivate.

Die nach dem erfindungsgemässen Verfahren erhältlichen Mono- und Polyazofarbstoffe werden in an sich bekannter Weise, vorteilhaft in Form ihrer Salze, insbesondere Alkali-, vor allem Natrium- oder Kaliumsalze, oder Ammoniumsalze, isoliert.

Die nach dem erfindungsgemässen Verfahren erhältlichen Farbstoffe stellen gut wasserlösliche anionische Farbstoffe dar, die allgemein für das Färben von mit anionischen Farbstoffen anfärbbaren textilen und nicht-textilen Substraten, z.B. für das Färben von Fasermaterialien aus natürlicher oder regenerierter Cellulose wie z.B. Baumwolle, synthetischen Polyamiden wie z.B. Nylon, Wolle, Seide, Polyurethanen oder basisch modifizierten Polyolefinen, und vorzugsweise für das Färben von Leder, geeignet sind.

Die DE-B-12 31 251 beschreibt bereits ein kontinuierliches Diazotierungsverfahren bei höherer Temperatur, jedoch nicht mit den Aminen und nicht mit einem Überschuss an Alkalinitrit gemäss der vorliegenden Anmeldung.

Die mit den nach dem erfindungsgemässen Verfahren hergestellen Farbstoffen erhältlichen Färbungen zeichnen sich durch gute applikatorische und färberische Eigenschaften, z.B. gute Licht-, Wasser-, Wasch-, Schweiss-, Trockenreinigungs-, Säure-, Alkali-, Lösungsmittel- und Diffusionsechtheit gegenüber Weich-PVC, gute Beständigkeit gegen über Elektrolyten wie Natrium- oder Calciumsalzen sowie gegenüber Eisen-, Chrom-, Kobalt- oder Kupfersalzen und gutes Aufbauvermögen auf Reinchromleder und auf nachgegerbtem Leder, aus.

Die nachfolgenden Beispiele dienen der Erläuterung der Erfindung, ohne sie darauf zu beschränken. Teile bedeuten Gewichtsteile und Prozente Gewichtsprozente.

Beispiel 1: 309 Teile 4'-Amino-4-nitrodiphenylamin-2-sulfosäure werden in 2700 Teilen Wasser bei etwa 70 °C angeschlämmt und durch Zugabe von ca. 90 Teilen 50 %iger NaOH-Lösung und Erhitzen auf ca. 80 °C vollständig in Lösung gebracht. Anschliessend wird die Aminlösung adiabatisch oder indirekt gekühlt und in einem kontinuierlichen Durchflussreaktor bei 47 - 50 °C diazotiert. Synchron mit der Aminlösung werden 424 Teile 32 %ige Salzsäure und 172 Teile einer 46 %igen wässrigen Natriumnitrit-Lösung in den Reaktor zudosiert. Der Reaktor ist mit einem intensiven Mischorgan für sehr gute Mikrovermischung ausgerüstet. Die Durchlaufzeit beträgt 0,9 Minuten. Beim Verlassen des Reaktors ist das eingesetzte Amin vollständig diazotiert.

Die resultierende Diazosuspension wird über einen indirekten Wärmeaustauscher auf ca. 35 °C abgekühlt und in einem Empfangskessel über Eis/Wasser-Gemisch in Gegenwart von ca. 3 Teilen Sulfaminsäure aufgefangen. Dabei sinkt die Temperatur der Diazomasse auf 0 °C. Das so gekühlte Diazoniumsalz wird anschliessend zu dem nach bekannten Methoden erhaltenen Kupplungsprodukt aus 170 Teilen diazotierter 1-Amino-8-naphthol-3,6-disulfosäure und 55 Teilen Resorcin in sodagepuffertem alkalischem Medium bei pH ca. 8,6 zugegeben. Die Reaktionsführung erfolgt adiabatisch, wobei die Temperatur, nach anfänglicher Kühlung des Reaktionsgemisches mit Eis auf 5 °C, frei auf 18 °C steigen kann. Der pH-Wert wird durch Zugabe von NaOH-Lösung konstant gehalten.

Der erhaltene Farbstoff wird auf übliche Weise durch Zugabe von NaCI in stark saurem Medium ausgesalzt, isoliert und getrocknet. Man erhält etwa 915 Teile trockenes Pulver, das Leder nach üblichen Färbemethoden in dunkelbraunen Tönen färbt.

Man erhält, bezogen auf 4'-Amino-4-nitrodiphenylamin-2-sulfosäure, eine um 12 - 15 % höhere Farbstoffausbeute als beim Arbeiten nach üblichen diskontinuierlichen Herstellungsmethoden, da praktisch keine Zersetzung der Diazokomponente vor der Kupplung stattfindet.

Der Farbstoff enthält keine mit bekannten Methoden feststellbaren sek.-NO-Gruppen mehr. Auch die Qualitätskonstanz des Farbstoffes ist überraschend hoch. Die Nuance schwankt nur noch geringfügig innerhalb der akzeptierten Standardkonformität, so dass kostspielige Korrekturmassnahmen in der Endstandardisierstufe nicht mehr nötig sind, die bei den bekannten Verfahren nicht zu umgehen sind, da dort die Nuance des erhaltenen Farbstoffes stark schwankt, auch bei nur minimalen, unvermeidbaren Abweichungen der Reaktionsbedingungen, wie Temperatur, pH-Wert, Rührgeschwindigkeit etc.

Beispiel 2:Auf analoge Weise wie im Beispiel 1 beschrieben, stellt man einen braunen Farbstoff her, indem man zuerst 4'-Amino-4-nitrodiphenylamin-2-sulfosäure diazotiert und mit der äquivalenten Menge 1-Aminonaphthalin-6-sulfosäure kuppelt, den erhaltenen Azofarbstoff diazotiert und auf die äquivalente Menge Resorcin kuppelt und schliesslich auf den erhaltenen Disazofarbstoff eine äquivalente Menge diazotierte 4'-Amino-4-nitrodiphenylamin-2-sulfosäure kuppelt, wobei die Diazotierung von 4'-Amino-4-nitrodiphenylamin-2-sulfosäure und die darauf folgende Kupplung jeweils wie im Beispiel 1 beschrieben erfolgen.

Man erhält den braunen Farbstoff in guter Ausbeute und bei verschiedenen Ansätzen mit konstanter Nuance.

Beispiel 3:Auf analoge Weise wie im Beispiel 1 beschrieben, stellt man einen braunen Farbstoff her, indem man zuerst 220 Teile Anilin-2,5-disulfosäure diazotiert und mit 670 Teilen einer Mischung, enthaltend hauptsächlich die Verbindungen, die im COLOUR INDEX die Colour Index Constitution Numbers, C.I. 75240 und 75660 tragen, kuppelt, auf das erhaltene Reaktionsprodukt anschliessend die Diazoniumverbindung aus 160 Teilen 4-Nitroanilin und danach die Diazoniumverbindung aus 230 Teilen 4'-Amino-4-nitrodiphenylamin-2-sulfosäure kuppelt, wobei die Diazotierung von 4'-Amino-4-nitrodiphenylamin-2-sulfosäure und die darauf folgende Kupplung wie im Beispiel 1 beschrieben erfolgen.

Man erhält den braunen Farbstoff in guter Ausbeute und bei verschiedenen Ansätzen mit konstanter Nuance.

Beispiel 4: Auf analoge Weise wie im Beispiel 1 beschrieben, stellt man einen braunen Farbstoff her, indem man zuerst 200 Teile 1-Aminonaphthalin-5-sulfosäure diazotiert und mit 450 Teilen einer Mischung, enthaltend hauptsächlich die Verbindungen, die im COLOUR INDEX die Colour Index Constitution Numbers, C.I. 75240 und 75660 tragen, kuppelt, auf das erhaltene Reaktionsprodukt anschliessend die Diazoniumverbindung aus 155 Teilen 4-Nitroanilin und danach die Diazoniumverbindung aus 230 Teilen 4'-Amino-4-nitrodiphenylamin-2-sulfosäure kuppelt, wobei die Diazotierung von 4'-Amino-4-nitrodiphenylamin-2-sulfosäure und die darauf folgende Kupplung wie im Beispiel 1 beschrieben erfolgen.

Man erhält den braunen Farbstoff in guter Ausbeute und bei verschiedenen Ansätzen mit konstanter Nuance.

Beispiel 5: Auf analoge Weise wie im Beispiel 1 beschrieben, stellt man einen braunen Farbstoff her, indem man zuerst 310 Teile 1-Hydroxy-8-aminonaphthalin-3,6-disulfosäure (diazotiert und mit 115 Teilen Resorcin kuppelt, auf das erhaltene Reaktionsprodukt anschliessend die Diazoniumverbindung aus 130 Teilen 4-Methoxyanilin und danach die Diazoniumverbindung aus 465 Teilen 4'-Amino-4-nitrodiphenylamin-2-sulfosäure kuppelt, wobei die Diazotierung von 4'-Amino-4-nitrodiphenylmin-2-sulfosäure und die darauf folgende Kupplung wie im Beispiel 1 beschrieben erfolgen.

Man erhält den braunen Farbstoff in guter Ausbeute und bei verschiedenen Ansätzen mit konstanter Nuance.

Beispiel 6: Auf analoge Weise wie im Beispiel 1 beschrieben, stellt man einen braunen Farbstoff her, indem man zuerst 590 Teile 4'-Amino-4-nitrodiphenylamin-2-sulfosäure diazotiert und mit 440 Teilen eines Gemisches aus 1 -Aminonaphthalin-6-sulfosäure und 1-Aminonaphthalin-7-sulfosäure kuppelt, das erhaltene Reaktionsprodukt anschliessend diazotiert und auf 160 Teile Phenol kuppelt und schliesslich das erhaltene Reaktionsprodukt mit 387 Teilen p-Toluolsulfosäure tosyliert, wobei die Diazotierung von 4'-Amino-4-nitrodiphenylamin-2-sulfosäure und die darauf folgende Kupplung wie im Beispiel 1 beschrieben erfolgen.

Man erhält den braunen Farbstoff in guter Ausbeute und bei verschiedenen Ansätzen mit konstanter Nuance.

Beispiel 7:Auf analoge Weise wie im Beispiel 1 beschrieben, stellt man einen braunen Farbstoff her, indem man zuerst 190 Teile 2-Hydroxy-3-nitroanilin-5-sulfosäure diazotiert und mit 100 Teilen Resorcin kuppelt, auf das erhaltene Reaktionsprodukt anschliessend die Diazoniumverbindung aus 100 Teilen 4'-Amino-4-nitrodiphenylamin-2-sulfosäure kuppelt, auf das so erhaltene Reaktionsprodukt anschliessend die Diazoniumverbindung aus 100 Teilen 4-Nitroanilin und schliesslich die Diazoniumverbindung aus 215 Teilen 4'-Amino-4-nitrodiphenylamin-2-sulfosäure kuppelt, wobei die Diazotierung von 4'-Amino-4-nitrodiphenylamin-2-sulfosäure und die darauf folgende Kupplung jeweils wie im Beispiel 1 beschrieben erfolgen. Das erhaltene Reaktionsprodukt wird danach noch mit Kupfersulfat auf übliche Weise in den Kupferkomplex überführt.

Man erhält den braunen Farbstoff in guter Ausbeute und bei verschiedenen Ansätzen mit konstanter Nuance.

## Patentansprüche

1. Verfahren zur Herstellung von Azofarbstoffen, ausgehend von einem Amin der Formel 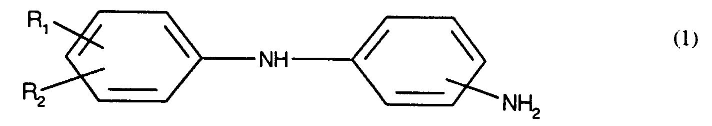 worin
R₁ Wasserstoff oder Nitro und
R₂ Wasserstoff, Sulfo, C₁-C₄-Alkyl oder C₁-C₄-Alkoxy bedeutet,
oder ausgehend von einem Amin. ausgewählt aus der Gruppe, bestehend aus 2-Aminobenzthiazol, 6-Nitro-2-aminobenzthiazol, 6-Benzoyl-2-aminobenzthiazol, 6-Methyl-2-aminobenzthiazol, 6-Methoxy-2-aminobenzthiazol, 2-Aminotriazol, 5-Methylsulfonyl-2-aminotriazol, 5-Nitro-2-aminotriazol, 3-Amino-1,2,4-triazol, 3-Aminoindazol, 5-Aminoindazol, 4-Aminochinolin und Aminobenzimidazol-Derivaten,
dadurch gekennzeichnet, dass man bei 35 bis 65 °C eine wässrige Lösung oder Suspension des Amins, eine wässrige Lösung eines Alkalinitrits und eine Mineralsäure kontinuierlich zu einer Disazoverbindung umsetzt, wobei man das Alkalinitrit in 3 bis 15 %igem Überschuss verwendet und die Säure in einer bei Diazotierungen in wässrigen verdünnten Mineralsäuren üblichen Konzentration einsetzt, und anschliessend die Diazoverbindung mit einer Kupplungskomponente kuppelt.

2. Verfahren gemäss Anspruch 1, dadurch gekennzeichnet, dass man ein Amin der Formel (1) diazotiert.

3. Verfahren gemäss Anspruch 1, dadurch gekennzeichnet, dass man ein Amin der Formel (1) diazotiert, worin R₁ Nitro, insbesondere Nitro in p-Stellung zur NH-Gruppe, bedeutet.

4. Verfahren gemäss einem der Ansprüche 2 oder 3, dadurch gekennzeichnet, dass man ein Amin der Formel (1) diazotiert, worin R₂ Wasserstoff oder vor allem Sulfo bedeutet.

5. Verfahren gemäss Anspruch 1, dadurch gekennzeichnet, dass man ein Amin der Formel 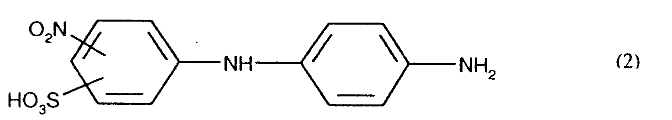 diazotiert.

6. Verfahren gemäss Anspruch 5. dadurch gekennzeichnet. dass man 4'-Amino-4-nitrodiphenylamin-2-sulfosäure diazotiert.

7. Verfahren gemäss einem der Ansprüche 1 bis 6, dadurch gekennzeichnet, dass man das zu diazotierende Amin als wässrige Lösung einsetzt, erhalten durch Anschlämmen des Amins in Wasser und Zugabe einer Base und Erhitzen auf 40 bis 90 °C.

8. Verfahren gemäss Anspruch 7. dadurch gekennzeichnet, dass man als Base KOH oder NaOH verwendet.

9. Verfahren gemäss einem der Ansprüche 1 bis 8, dadurch gekennzeichnet, dass man als Alkalinitrit Kalium- oder Natriumnitrit verwendet.

10. Verfahren gemäss einem der Ansprüche 1 bis 9, dadurch gekennzeichnet, dass man das Alkalinitrit in 5 bis 10 %igem Überschuss, bezogen auf das Amin, verwendet.

11. Verfahren gemäss einem der Ansprüche 1 bis 10, dadurch gekennzeichnet, dass man die Diazotierung bei einer Temperatur zwischen 40 und 55 °C durchführt.

12. Verfahren gemäss einem der Ansprüche 1 bis 11, dadurch gekennzeichnet, dass die Verweilzeit in dem Durchflussreaktor zwischen 0,1 und 10 Minuten, vorzugsweise zwischen 0,5 und 5 Minuten, liegt.

## Claims

1. A process for the preparation of an azo dye, starting from an amine of formula 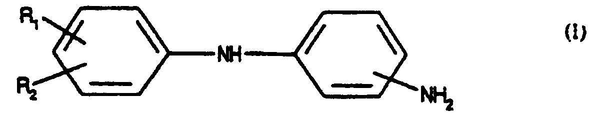 wherein
R₁ is hydrogen or nitro, and
R₂ is hydrogen, sulfo, C₁-C₄alkyl or C₁-C₄alkoxy,
or starting from an amine selected from the group consisting of 2-aminobenzothiazole, 6-nitro-aminobenzothiazole, 6-benzoyl-2-aminobenzothiazole, 6-methyl-2-aminobenzothiazole, 6-methoxy-2-aminobenzothiazole, 2-aminotriazole, 5-methylsulfonyl-2-aminotriazole, 5-nitro-2-aminotriazole, 3-amino-1,2,4-triazole, 3-aminoindazole, 5-aminoindazole, 4-aminoquinoline and aminobenzimidazole derivatives,
which comprises reacting an aqueous solution or suspension of the amine, an aqueous solution of an alkali metal nitrite and a mineral acid continuously at from 35 to 65°C to give a disazo compound, using a 3 to 15% excess of the alkali metal nitrite and using the acid at a concentration customary for diazotisations in dilute aqueous mineral acids, and subsequently coupling the diazo compound to a coupling component.

2. A process according to claim 1, which comprises diazotising an amine of formula (1).

3. A process according to claim 1, which comprises diazotising an amine of formula (1) wherein R₁ is nitro, preferably nitro in para-position to the NH group.

4. A process according to one of claims 2 or 3, which comprises diazotising an amine of formula (1) wherein R2 is hydrogen or, preferably, sulfo.

5. A process according to claim 1, which comprises diazotising an amine of formula

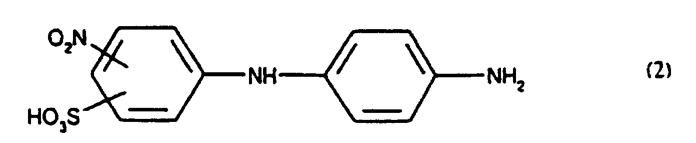

6. A process according to claim 5, which comprises diazotising 4'-amino-4-nitrodiphenyl-amine-2-sulfonic acid.

7. A process according to any of claims 1 to 6, which comprises using the amine to be diazotised as an aqueous solution obtained by suspending the amine in water and adding a base and heating at from 40 to 90°C.

8. A process according to claim 7, wherein the base is KOH or NaOH.

9. A process according to one of claims 1 to 8, wherein the alkali metal nitrite is potassium or sodium nitrite.

10. A process according to one of claims 1 to 9, wherein a 5 to 10% excess of the alkali metal nitrite is used, based on the amine.

11. A process according to one of claims 1 to 10, wherein the diazotisation is carried out at a temperature between 40 and 55°C.

12. A process according to any one of claims 1 to 11, wherein the residence time in the flow-through reactor is between 0.1 and 10 minutes, preferably between 0.5 and 5 minutes.

## Revendications

1. Procédé pour la préparation de colorants azoïques, en partant d'une amine de formule 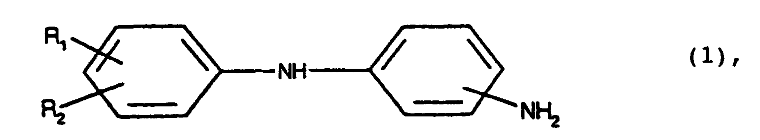 où
R₁ représente un atome d'hydrogène ou un groupe nitro
et
R₂ représente un atome d'hydrogène, des groupes sulfo, alkyle en C₁-C₄ ou alkoxy en C₁-C₄,
ou en partant d'une amine prise dans le groupe comportant des dérivés de 2-aminobenzothiazole, de 6-nitro-2-amino-benzothiazole, de 6-benzoyl-2-aminobenzothiazole, de 6-méthyl-2-aminobenzothiazole, de 6-méthoxy-2-amino-benzothiazole, de 2-aminotriazole, de 5-méthyl-sulfonyl-2-aminotriazole, de 5-nitro-2-aminotriazole, de 3-amino-1,2,4-triazole, de 3-aminoindazole, de 5-aminoindazole, de 4-aminoquinoléine et d'aminobenzimidazole,
caractérisé en ce qu'on fait réagir, à une température de 35 à 65°C, une solution ou suspension aqueuse de l'amine, une solution aqueuse d'un nitrite alcalin et un acide minéral en continu pour obtenir un composé disazoïque, en utilisant le nitrite alcalin en un excès de 3 à 15 % et l'acide en une concentration usuelle dans la diazotation en milieu d'acides minéraux aqueux, dilués, et ensuite en copulant le composé diazoïque avec un composant de copulation.

2. Procédé selon la revendication 1, caractérisé en ce qu'on diazote une amine de formule (1).

3. Procédé selon la revendication 1, caractérisé en ce qu'on diazote une amine de formule (1), où R₁ représente un groupe nitro, en particulier un groupe nitro en position para par rapport au groupe NH.

4. Procédé selon l'une des revendications 2 ou 3, caractérisé en ce qu'on diazote une amine de formule (1), où R₂ représente un atome d'hydrogène ou avant tout un groupe sulfo.

5. Procédé selon la revendication 1, caractérisé en ce qu'on diazote une amine de formule (2)

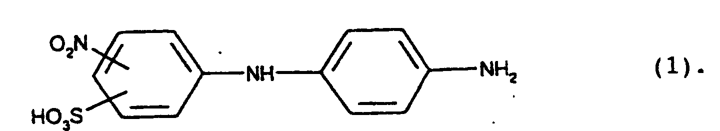

6. Procédé selon la revendication 5, caractérisé en ce qu'on diazote l'acide 4'-amino-4-nitrodiphénylamino-2-sulfonique.

7. Procédé selon l'une des revendications 1 à 6, caractérisé en ce qu'on utilise l'amine à diazoter sous forme de solution aqueuse obtenue par mise en suspension de l'amine dans l'eau et par ajout d'une base et chauffage à une température de 40 à 90°C.

8. Procédé selon la revendication 7, en ce qu'on utilise KOH ou NaOH en tant que base.

9. Procédé selon l'une des revendications 1 à 8, caractérisé en ce qu'on utilise le nitrite de sodium ou de potassium en tant que nitrite alcalin.

10. Procédé selon l'une des revendications 1 à 9, caractérisé en ce qu'on utilise le nitrite alcalin dans un excès de 5 à 10 %, par rapport à l'amine.

11. Procédé selon l'une des revendications 1 à 10, caractérisé en ce qu'on met en oeuvre la diazotation à une température comprise entre 40 et 55°C.

12. Procédé selon l'une des revendications 1 à 11, caractérisé en ce que la durée de séjour dans le réacteur à passage est comprise entre 0,1 et 10 minutes, de préférence entre 0,5 et 5 minutes.
